# EUROPEAN PATENT APPLICATION

(11) **EP 4 349 807 A1**
(43) Date of publication of application: **10.04.2024**
(21) Application number: 22816110.5
(22) Date of filing: 31.05.2022
(51) Int. Cl.: C07C 51/43, B01D 9/02, C07C 57/055

(54) **METHOD FOR PRODUCING EASILY POLYMERIZABLE COMPOUND**

(30) Priority: 02.06.2021 JP 2021093027
(71) Applicant: Nippon Shokubai Co., Ltd., Osaka-shi, Osaka 541-0043 (JP)
(72) Inventor: MATSUDA, Takayuki, Himeji-shi, Hyogo 671-1282 (JP); MUKAE, Masashi, Himeji-shi, Hyogo 671-1282 (JP)
(74) Representative: Schön, Christoph
(86) International application number: PCT/JP2022/022139
(87) International publication number: WO 2022/255364

(57) **Abstract**

The present invention provides a method for efficiently obtaining a high-quality product. The present invention relates to a method for producing an easily polymerizable compound, the method using a purification apparatus including a wash column and at least one of a crystallization tank or a ripening tank, the crystallization tank forming easily polymerizable compound crystals from an easily polymerizable compound-containing solution or a mother liquor in an easily polymerizable compound crystal-containing slurry, the ripening tank growing the easily polymerizable compound crystals, the purification apparatus further including a line that includes a bend having a ratio R/D of radius R of curvature to line inner diameter D of more than 4 and not more than 10 and that is connected to at least one selected from the group consisting of the crystallization tank, the ripening tank, and the wash column, the method including sending the easily polymerizable compound crystal-containing slurry through the line.

## Description

### TECHNICAL FIELD

The present invention relates to a method for producing easily polymerizable compounds. Specifically, the present invention relates to a method for producing an easily polymerizable compound, a method for purifying an easily polymerizable compound, and an apparatus for purifying an easily polymerizable compound.

### BACKGROUND ART

Purification apparatuses have been widely used industrially to purify compounds that are used as raw materials for resins, for example. In many fields of the chemical industry, high-quality compounds with reduced impurities have been required, and for such compounds, various better purification apparatuses have been studied.

Industrially, many of crude compounds, which are compounds before purification, are purified through continuous purification. In continuous purification, the apparatuses are connected with a pipe to constitute a line that enables transport between the apparatuses (hereinafter, the "line" is referred to as a pipe(s) in a connected state or to a state where connection is formed by a pipe(s)), and liquid to be purified is transported through the line. For example, a method for producing acrylic acid, which is an easily polymerizable compound, has been suggested in which a raw material gas is subjected to a catalytic vapor phase oxidation reaction, the acrylic acid solution obtained by collecting is fed through a line to a crystallization apparatus for purification, the residual mother liquor is fed through a line to a distillation reactor, acrylic acid obtained by decomposing a substance obtained by Michael addition of acrylic acid is returned through a line to the collecting step (see, for example, Patent Literature 1).

Regarding transport of a solid-containing slurry, when catalysts such as ion exchange resins used in the production of unsaturated carboxylic acid esters are recycled, the shape of a pipe that transports a slurry containing resin (catalyst) as solids, the resin concentration of the slurry, and the flow velocity of the slurry are specified. Thereby, stagnation of the slurry in the pipe and fracture of the resin can be prevented (see, for example, Patent Literature 2).

### CITATION LIST

### - Patent Literature

Patent Literature 1: JP 2007-182437 A
Patent Literature 2: JP 2002-338520 A

### SUMMARY OF INVENTION

### - Technical Problem

As described above, better methods for producing easily polymerizable compounds have been desired, and methods for efficiently obtaining high-quality products (easily polymerizable compounds) have been desired. The present invention has been made in view of the above-mentioned current state of the art, and aims to provide a method for efficiently obtaining a high-quality product.

### - Solution to Problem

The present inventors have investigated a method for efficiently obtaining a high-quality product, focused on a continuous purification apparatus that enables efficient purification, and studied on a purification apparatus including a wash column and at least one of a crystallization tank or a ripening tank, the crystallization tank forming easily polymerizable compound crystals from an easily polymerizable compound-containing solution or a mother liquor in an easily polymerizable compound crystal-containing slurry, the ripening tank growing the easily polymerizable compound crystals. The present inventors found that when a crystal-containing slurry is sent through a line in such a purification apparatus in production of an easily polymerizable compound, the concentration of impurities in the product may be high depending on the conditions of the line. In this respect, the present inventors intensively studied and focused as described above on a method for producing an easily polymerizable compound using a purification apparatus that includes a line that includes a bend having a ratio R/D of radius R of curvature to line inner diameter D of more than 4 and not more than 10 and that is connected to at least one selected from the group consisting of the crystallization tank, the ripening tank, and the wash column, the method including sending the easily polymerizable compound crystal-containing slurry through the line. The present inventors found that this production method can provide a high-quality product and can sufficiently prevent clogging of the line by crystals, solving the above problems. Thus, the present invention has been completed.

The reason why a high-quality product can be obtained by the above-described production method is presumably that in the line including a gently curved bend, uneven crystal growth and crystal fracture are prevented to sufficiently prevent widening of the particle size distribution of the crystals, so that a crystal bed having a substantially uniform crystal shape can be formed in the wash column and the washability thereof is improved.

That is, the present invention relates to a method for producing an easily polymerizable compound, the method using a purification apparatus including a wash column and at least one of a crystallization tank or a ripening tank, the crystallization tank forming easily polymerizable compound crystals from an easily polymerizable compound-containing solution or a mother liquor in an easily polymerizable compound crystal-containing slurry, the ripening tank growing the easily polymerizable compound crystals,
the purification apparatus further including a line that includes a bend having a ratio R/D of radius R of curvature to line inner diameter D of more than 4 and not more than 10 and that is connected to at least one selected from the group consisting of the crystallization tank, the ripening tank, and the wash column,
the method including sending the easily polymerizable compound crystal-containing slurry through the line.

Patent Literature 2 only discloses transport of a slurry in which the solids are resin (catalyst), and does not disclose anything about transport of an easily polymerizable compound crystal-containing slurry or about obtaining a high-quality product.

### - Advantageous Effects of Invention

The production method of the present invention can efficiently provide a high-quality product.

### BRIEF DESCRIPTION OF DRAWINGS

FIG. 1 is a schematic view of an exemplary bend of a line used in a production method of the present invention.
FIG. 2 is a schematic diagram of exemplary lines to which the present invention is applicable, in a purification apparatus of the present invention.
FIG. 3 is a schematic diagram of other exemplary lines to which the present invention is applicable, in the purification apparatus of the present invention.
FIG. 4 is a schematic diagram of other exemplary lines to which the present invention is applicable, in the purification apparatus of the present invention.
FIG. 5 is a schematic diagram of other exemplary lines to which the present invention is applicable, in the purification apparatus of the present invention.
FIG. 6 is a schematic diagram of other exemplary lines to which the present invention is applicable, in the purification apparatus of the present invention.
FIG. 7 is a schematic diagram of other exemplary lines to which the present invention is applicable, in the purification apparatus of the present invention.
FIG. 8 is a schematic diagram of other exemplary lines to which the present invention is applicable, in the purification apparatus of the present invention.

### DESCRIPTION OF EMBODIMENTS

The present invention is described in detail below.

It should be noted that combinations of two or more of the preferred features of the present invention described below are also preferred embodiments of the present invention.

The following first describes a method for producing an easily polymerizable compound of the present invention, followed by descriptions of a method for purifying an easily polymerizable compound of the present invention and a purification apparatus of the present invention in this order.

### (Method for producing easily polymerizable compound of the present invention)

The production method of the present invention relates to a method for producing an easily polymerizable compound, the method using a purification apparatus including a wash column and at least one of a crystallization tank or a ripening tank, the crystallization tank forming easily polymerizable compound crystals from an easily polymerizable compound-containing solution or a mother liquor in an easily polymerizable compound crystal-containing slurry, the ripening tank growing the easily polymerizable compound crystals, the purification apparatus further including a line that includes a bend having a ratio R/D of radius R of curvature to line inner diameter D of more than 4 and not more than 10 and that is connected to at least one selected from the group consisting of the crystallization tank, the ripening tank, and the wash column, the method including sending the easily polymerizable compound crystal-containing slurry through the line.

The following first describes the sending the slurry, followed by description of other steps. In the case of continuous purification, the steps are typically performed simultaneously when the purification apparatus is viewed as a whole.

Herein, the term "easily polymerizable compound" refers to an easily polymerizable compound obtainable by the production method of the present invention, and does not refer to raw materials, by-products, or solvents in the production method of the present invention. The term "easily polymerizable compound" may also be referred to as a "target compound" or a "target substance". Herein, the term "impurities" refers to components other than the "easily polymerizable compound", such as raw materials, by-products, and solvents.

Herein, the phrase "forming easily polymerizable compound crystals from an easily polymerizable compound-containing solution or a mother liquor in an easily polymerizable compound crystal-containing slurry" encompasses not only forming easily polymerizable compound crystals from an easily polymerizable compound-containing solution but also newly forming crystals from a mother liquor in an easily polymerizable compound crystal-containing slurry so that the slurry has a higher crystal concentration after being discharged from the crystallization tank than before being fed to the crystallization tank.

For example, the method for producing an easily polymerizable compound of the present invention, the method for purifying an easily polymerizable compound of the present invention, and the purification apparatus of the present invention encompass not only those in which an easily polymerizable compound-containing solution is fed to the crystallization tank to provide an easily polymerizable compound crystal-containing slurry but also those in which an easily polymerizable compound crystal-containing slurry is fed to the crystallization tank to provide a slurry having a higher crystal concentration. The easily polymerizable compound crystal-containing slurry to be fed to the crystallization tank typically contains an easily polymerizable compound also in its mother liquor.

When the crystals are discharged from the crystallization tank in the present invention, they are typically discharged in the form of an easily polymerizable compound crystal-containing slurry.

### <Sending easily polymerizable compound crystal-containing slurry>

In the sending the slurry in the production method of the present invention, an easily polymerizable compound crystal-containing slurry is sent in the purification apparatus through a line that includes a bend that has a ratio R/D of radius R of curvature to line inner diameter D of more than 4 and not more than 10 and that is connected to at least one selected from the group consisting of the crystallization tank, the ripening tank, and the wash column.

The radius R of curvature is the reciprocal of the curvature of the bend of the line, and corresponds to the radius of the circle including the centerline of the bend of the line as an arc. The larger the radius R of curvature, the gentler the curve of the bend.

When the ratio R/D of radius R of curvature to line inner diameter D is more than 4, a high-quality easily polymerizable compound can be obtained. This is presumably because the curve of the bend is gentle, and in the line, uneven crystal growth and crystal fracture are prevented to sufficiently prevent widening of the particle size distribution of the crystals, so that a crystal bed having a substantially uniform crystal shape can be formed in the wash column. Also, clogging of the line by crystals can be sufficiently prevented.

The ratio R/D of radius R of curvature to line inner diameter D is preferably 4.5 or more, more preferably 5 or more.

In the production method of the present invention, the ratio R/D of radius R of curvature to line inner diameter D is 10 or less. Thereby, the line can be suitably installed without excessively increasing the scale of the purification apparatus. From the viewpoint of the scale of the purification apparatus, the ratio R/D of radius R of curvature to line inner diameter D is preferably 9 or less, more preferably 8 or less.

The radius R of curvature is not limited and is preferably, for example, 50 to 5000 mm, more preferably 100 to 2500 mm, in an industrial-scale purification apparatus.

The line inner diameter D is preferably, for example, 10 to 500 mm, more preferably 20 to 250 mm, in an industrial-scale purification apparatus.

The bend is not limited, and the angle of the bend (the angle formed by the line portions extending forward and backward from the bend) is preferably 75° to 165°, more preferably 90° to 150°.

The line is connected to at least one selected from the group consisting of the crystallization tank, the ripening tank, and the wash column.

Examples of the line include a line that connects crystallization tank(s), a line that connects a crystallization tank to a ripening tank, a line that connects a crystallization tank or a ripening tank to a wash column, and a line that connects a wash column to a melting unit in a melt loop which is described later. The purification apparatus may include one or more of these lines. The line that connects crystallization tank(s) may be a line that connects a slurry discharge port to a slurry feed port in one crystallization tank. When multiple tanks (crystallization tanks) are connected in series, the line may connect a downstream tank to an upstream tank. A different unit may be installed in the middle of the line. For example, an out-of-tank cooling mechanism or a solid-liquid separator for crystallization may be installed in the middle of the line that connects the slurry discharge port to the slurry feed port.

The line is preferably a line that connects crystallization tank(s), a line that connects a crystallization tank to a ripening tank, or a line that connects a crystallization tank or a ripening tank to a wash column.

In particular, more preferably, in the production method of the present invention, the line connects a crystallization tank or a ripening tank to a wash column, and in the sending the slurry, the line sends the easily polymerizable compound crystal-containing slurry from the crystallization tank or the ripening tank to the wash column.

When the ratio R/D of the bend of the line is within the range of R/D in the present invention, a crystal bed having a substantially uniform crystal shape can be formed in the wash column. Thereby, a higher-quality product can be obtained.

For example, when the purification apparatus of the present invention includes multiple tanks connected in series, the crystallization tank or the ripening tank to which the line is connected may be the tank arranged last in series (the crystallization tank or the ripening tank).

The line may be made of any material and is preferably made of stainless steel or another metal or an alloy, for example.

Preferably, in the sending the slurry in the production method of the present invention, the linear velocity of the slurry is 1.5 m/s or more and 4.0 m/s or less.

When the linear velocity of the slurry is 1.5 m/s or more, uneven crystal growth can be further prevented during sending the slurry through the line including a bend in the present invention. Also, clogging of the line by crystals can be further prevented.

The linear velocity of the slurry is more preferably 1.8 m/s or more, still more preferably 2.0 m/s or more.

When the linear velocity of the slurry is 4.0 m/s or less, fracture of the crystals in the slurry can be further prevented during sending the slurry through the line including a bend in the present invention. The linear velocity of the slurry is more preferably 3.5 m/s or less, still more preferably 3.0 m/s or less.

In other words, when the linear velocity of the slurry is 1.5 m/s or more and 4.0 m/s or less, uneven crystal growth and clogging and fracture of crystals in the line can be further prevented. These remarkable effects can be obtained at the same time.

The linear velocity of the slurry can be determined by directly measuring the volume flow rate in the straight pipe section with a flow meter or by measuring the mass flow rate in the straight pipe section with a flow meter, dividing the measured value by the slurry density to calculate a volume flow rate, and dividing the volume flow rate by the cross-sectional area of the bend through which the slurry flows.

Preferably, in the sending the slurry in the production method of the present invention, the crystal concentration of the slurry is 1% by mass or higher and 40% by mass or lower.

When the crystal concentration of the slurry is 1% by mass or higher, a suitable flow rate of the slurry can be achieved and the product can be obtained efficiently without increasing the size of units such as pumps. The crystal concentration of the slurry is more preferably 5% by mass or higher, still more preferably 10% by mass or higher.

When the crystal concentration of the slurry is 40% by mass or lower, the slurry can have sufficient fluidity and clogging of the line can be sufficiently prevented during sending the slurry through the line including a bend in the present invention. The crystal concentration of the slurry is more preferably 38% by mass or lower, still more preferably 35% by mass or lower.

Preferably, in the production method of the present invention, the line includes a heating mechanism.

Examples of the heating mechanism include a jacket including a heat retaining mechanism and using a heating medium such as hot water, cooling water (CW), or brine, a steam tracing system, and an electric heater. One or more of these can be used.

The heating temperature by the heating mechanism may be appropriately set in consideration of the melting point of the easily polymerizable compound and the like. For example, preferably, in the production method of the present invention, the line includes a heating mechanism and in the sending the slurry, the slurry temperature is 1°C or higher and 50°C or lower.

Preferably, the temperature difference between the heating temperature by the heating mechanism and the slurry temperature is 1°C to 40°C.

When the temperature difference is 1°C or more, uneven crystal growth in the line and clogging of the line caused by freezing can be further prevented during sending the slurry through the line including a bend in the present invention. The temperature difference is more preferably 2°C or more, still more preferably 3°C or more.

When the temperature difference is 40°C or less, melting of part of the crystals in the slurry can be prevented to further prevent widening of the particle size distribution during sending the slurry through the line including a bend in the present invention. The temperature difference is more preferably 30°C or less, still more preferably 20°C or less, particularly preferably 10°C or less.

The pressure condition in the line in the sending the slurry may be increased pressure, atmospheric pressure, or reduced pressure.

The sending the slurry in the production method of the present invention may be intermittent, and is preferably basically continuous.

The wash column in the purification apparatus of the present invention is not limited as long as it can wash the crystals, and is preferably one that forcibly transports the crystal bed. Specific examples of the wash column that forcibly transports the crystal bed include: a mechanical wash column in which the crystals are compacted with a piston to form a crystal bed and the crystal bed is transported; and a hydraulic wash column in which the slurry is sent to the column using a pump, a mother liquor is withdrawn through a filter in the column to form a crystal bed and the crystal bed is transported.

The wash column is more preferably a hydraulic wash column because of its high production capacity and because the wash column includes a small number of driving parts, leading to a reduction in troubles caused by the apparatus.

The wash column may include a mechanical mechanism that scrapes off the crystal bed, for example.

In the production method of the present invention, the easily polymerizable compound is preferably an easily polymerizable compound having a reactive double bond.

In particular, in the production method of the present invention, the easily polymerizable compound is more preferably an unsaturated carboxylic acid, still more preferably a (meth)acrylic acid, particularly preferably acrylic acid. Herein, the term "(meth)acrylic acid" refers to acrylic acid and/or methacrylic acid.

The easily polymerizable compound-containing solution described above is not limited to one synthesized in-house, and may be one procured from outside sources.

### <Crystallizing>

The production method of the present invention may include crystallizing an easily polymerizable compound-containing solution or an easily polymerizable compound crystal-containing slurry. The crystallizing can form easily polymerizable compound crystals from an easily polymerizable compound-containing solution or a mother liquor in an easily polymerizable compound crystal-containing slurry.

The temperature in the crystallizing may be appropriately adjusted according to the type of the easily polymerizable compound to be purified. The temperature is lower than the melting point of the pure substance by approximately 2°C to 15°C. When the easily polymerizable compound to be purified is a (meth)acrylic acid, the temperature is preferably 0°C to 12°C, more preferably 1°C to 11°C, still more preferably 2°C to 10°C.

The temperature in the crystallizing is the temperature of the easily polymerizable compound-containing solution or the mother liquor in the easily polymerizable compound crystal-containing slurry to be subjected to the crystallizing.

The pressure condition in the crystallizing may be increased pressure, atmospheric pressure, or reduced pressure.

The crystallizing is not limited as long as it forms the easily polymerizable compound crystals, and may be continuous crystallization or batch crystallization, with continuous crystallization being preferred.

The crystallizing can be performed using a crystallization tank and/or a ripening tank, which are described later.

The easily polymerizable compound crystal-containing slurry is preferably discharged from a portion in the vicinity of the bottom of the tank.

In the production method of the present invention, the easily polymerizable compound-containing solution is preferably a crude (meth)acrylic acid aqueous solution or a crude (meth)acrylic acid solution.

The crude (meth)acrylic acid aqueous solution refers to a solution in which a (meth)acrylic acid is dissolved in water and which contains impurities such as by-products produced during the production of the (meth)acrylic acid. The crude (meth)acrylic acid solution refers to a solution containing a (meth)acrylic acid and impurities such as by-products produced during the production of the (meth)acrylic acid.

Examples of the impurities include acids such as propionic acid, acetic acid, maleic acid, benzoic acid, and acrylic acid dimers; aldehydes such as acrolein, furfural, formaldehyde, and glyoxal; acetone; methyl isobutyl ketone; toluene; and protoanemonin.

The production method of the present invention can sufficiently remove impurities (e.g., in particular, furfural) in the easily polymerizable compound-containing solution or the easily polymerizable compound crystal-containing slurry. Thereby, a high-quality product can be obtained. Herein, the term "high-quality product" refers to any product in which at least one of the above-described impurities (e.g., furfural) is sufficiently reduced.

For example, the amount of furfural in the product of the easily polymerizable compound obtained by the production method of the present invention is preferably 10 ppm or less, more preferably 5 ppm or less, particularly preferably 0.2 ppm or less.

### <Feeding easily polymerizable compound-containing solution or easily polymerizable compound crystal-containing slurry to crystallization tank or ripening tank>

The production method of the present invention may include feeding an easily polymerizable compound-containing solution or an easily polymerizable compound crystal-containing slurry to a crystallization tank or a ripening tank.

Examples of the easily polymerizable compound-containing solution or easily polymerizable compound crystal-containing slurry to be fed to the tank include those containing the easily polymerizable compound, an aqueous solution of the easily polymerizable compound, and an aqueous slurry containing crystals of the easily polymerizable compound. The easily polymerizable compound-containing solution or the mother liquor in the easily polymerizable compound crystal-containing slurry typically contains impurities other than the easily polymerizable compound and water.

In the production method of the present invention, the purity (mass percentage) of the easily polymerizable compound in the easily polymerizable compound-containing solution or the mother liquor in the easily polymerizable compound crystal-containing slurry is preferably 99% by mass or lower.

The mass percentage of the easily polymerizable compound in the easily polymerizable compound-containing solution or the mother liquor in the easily polymerizable compound crystal-containing slurry is preferably 60% by mass or higher.

When the easily polymerizable compound-containing solution or the easily polymerizable compound crystal-containing slurry is fed to the crystallization tank or the ripening tank, the feed rate is not limited and is, for example, 0.2 × 10³ to 4.0 × 10⁵ kg/h in the case of an industrial-scale crystallization tank or ripening tank.

### <Recovering easily polymerizable compound from mother liquor separated by crystallizing>

The production method of the present invention may further include recovering the easily polymerizable compound from the mother liquor separated by the crystallizing.

The mother liquor to be used in the recovering can be obtained as follows: a supernatant liquid of the easily polymerizable compound crystal-containing slurry in the crystallization tank or the ripening tank is discharged and crystals are separated therefrom. The mother liquor typically contains an easily polymerizable compound. In other words, it is an easily polymerizable compound-containing solution.

The discharged mother liquor can be recovered in the recovery and reused. The recovered easily polymerizable compound may be reused, for example, by feeding to a tank or unit on the downstream (preceding stage) side so that the easily polymerizable compound in the mother liquor separated by the crystallizing can be reduced.

Non-limiting examples of the recovery include distillation. The mother liquor may be discharged using a pump or the like.

### <Agitating in ripening tank>

The production method of the present invention may include agitating the easily polymerizable compound crystal-containing slurry in the ripening tank.

In the agitating, the crystal-containing slurry is agitated typically using an agitator in the ripening tank.

### <Feeding slurry to wash column>

The production method of the present invention may include discharging the easily polymerizable compound crystal-containing slurry from the crystallization tank or the ripening tank and feeding the slurry to the wash column.

The feeding the slurry to the wash column corresponds to the sending the easily polymerizable compound crystal-containing slurry when the line that sends the slurry connects the crystallization tank or the ripening tank to the wash column.

In the feeding the slurry to the wash column, the temperature at which the crystal-containing slurry is sent can be appropriately selected according to the melting point of the easily polymerizable compound or the like, and can be appropriately adjusted within the range of 1°C to 50°C, for example.

For example, when the easily polymerizable compound is a (meth)acrylic acid, the temperature at which the crystal-containing slurry is sent is preferably 5°C to 13°C, more preferably 7°C to 11.5°C.

The temperature at which the crystal-containing slurry is sent is the temperature of the mother liquor in the crystal-containing slurry immediately before being sent to the wash column (e.g., the crystal-containing slurry in the pipe or nozzle that sends the crystal-containing slurry to the wash column).

### <Obtaining easily polymerizable compound-containing solution from raw material>

The production method of the present invention preferably includes obtaining an easily polymerizable compound-containing solution from a raw material.

The obtaining an easily polymerizable compound-containing solution from a raw material preferably includes obtaining an easily polymerizable compound-containing gas from a raw material and obtaining an easily polymerizable compound-containing solution from the easily polymerizable compound-containing gas.

The obtaining an easily polymerizable compound-containing gas from a raw material may be any process that can provide the easily polymerizable compound-containing gas. When the easily polymerizable compound is a (meth)acrylic acid, the obtaining an easily polymerizable compound-containing gas from a raw material can be more suitably performed by synthesis (catalytic vapor phase oxidation reaction) of acrylic acid as described in JP 2007-182437 A (Patent Literature 1).

The easily polymerizable compound-containing solution may be obtained from the easily polymerizable compound-containing gas by any process that can provide the easily polymerizable compound-containing solution. When the easily polymerizable compound is a (meth)acrylic acid, the easily polymerizable compound-containing solution can be suitably obtained by collecting acrylic acid as described in Patent Literature 1, for example.

In the production method of the present invention, the (meth)acrylic acid is preferably produced from at least one raw material selected from the group consisting of propane, propylene, acrolein, isobutene, methacrolein, acetic acid, lactic acid, isopropanol, 1,3-propanediol, glycerol, and 3-hydroxypropionic acid. The (meth)acrylic acid and/or the raw material may also be bio-based (meth)acrylic acids derived from renewable raw materials.

In the obtaining an easily polymerizable compound-containing gas, impurities such as by-products are basically generated. For example, when the easily polymerizable compound is a (meth)acrylic acid, the impurities generated include water; acids such as propionic acid, acetic acid, maleic acid, benzoic acid, and acrylic acid dimers; aldehydes such as acrolein, furfural, formaldehyde, and glyoxal; acetone; methyl isobutyl ketone; toluene; and protoanemonin. Such impurities (e.g., in particular, furfural) can be separated with excellent efficiency by the production method of the present invention. Thereby, a product can be efficiently obtained.

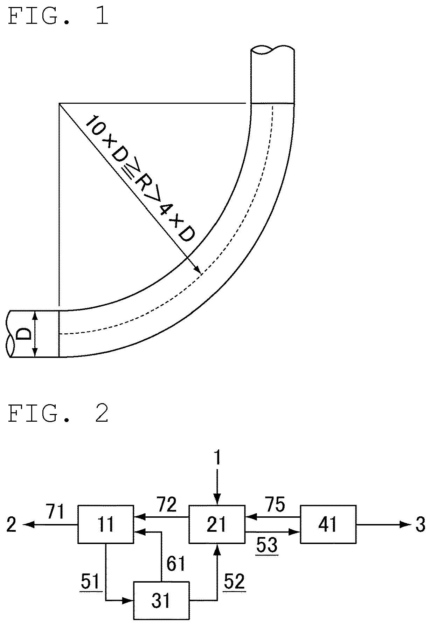

FIG. 1 is a schematic view of an exemplary bend of a line used in the production method of the present invention.

As shown in FIG. 1, the radius R of curvature described above corresponds to the radius of the circle in which the centerline of the bend of the line (curve extending along the center of the line inner diameter D) is an arc.

The line inner diameter D refers to the inner diameter of the bend of the line.

When the bend of the line used in the production method of the present invention has a ratio R/D of radius R of curvature to line inner diameter D of more than 4, the curve of the bend is gentle and a high-quality product can be efficiently obtained to achieve the effects of the present invention. When the bend has a ratio R/D of 10 or less, the line can be suitably installed without excessively increasing the scale of the purification apparatus.

FIGS. 2 to 8 are each a schematic diagram of exemplary lines to which the present invention is applicable, in the purification apparatus of the present invention.

In FIGS. 2 to 8, the present invention can be applied to the lines each connected to at least one selected from the group consisting of the crystallization tank, the ripening tank, and the wash column to which the easily polymerizable compound crystal-containing slurry is to be fed. In FIGS. 2 to 8, the lines with underlined numbers correspond to the lines to which the present invention is applicable.

In the present invention, each line connected to at least one selected from the group consisting of the crystallization tank, the ripening tank, and the wash column to which the easily polymerizable compound crystal-containing slurry is to be fed should have at least one bend satisfying the above-described ratio R/D of radius R of curvature to line inner diameter D. Preferably, all of the bends of each line satisfy the ratio R/D.

The following describes the details of the embodiments of the use of the purification apparatuses of the present invention shown in FIGS. 2 to 8.

FIG. 2 illustrates an apparatus including a crystallization unit including one crystallization tank and one ripening tank. The apparatus includes a line that directly sends a mother liquor from the ripening tank to the next downstream crystallization tank, and a line that directly discharges the residue (mother liquor) from the crystallization tank, which is the most downstream tank.

A compound solution 1 to be fed to the purification apparatus is introduced into a ripening tank 21. A slurry containing crystals precipitated through cooling in a crystallization tank 11 that includes a cooling mechanism is sent to a solid-liquid separator 31 through a line 51. In the solid-liquid separator 31, the slurry is separated into a mother liquor and a concentrated crystal slurry. The concentrated crystal slurry is sent to the adjacent ripening tank 21 through a line 52, and the mother liquor is returned to the crystallization tank 11 through a line 61. The residue 2 is discharged from the crystallization tank 11 through a line 71 to the outside of the purification apparatus. Thereby, the liquid level in the crystallization tank 11 is adjusted. The crystals are grown in the ripening tank 21, and then, the crystal slurry is sent to a mechanical wash column 41 through a line 53. In order to adjust the liquid level in the ripening tank 21, the mother liquor is directly sent from the ripening tank 21 to the crystallization tank 11 through a line 72.

In the mechanical wash column 41, the crystals are compacted with a piston to form a crystal bed. The crystal bed is scraped off in the lower part of the column, suspended in a circulation liquid, and melted by heating to obtain a melt. A portion of the circulation liquid containing the melt is sent out as a high-purity compound 3. A portion of the rest of the circulation liquid (washing liquid) is returned to the mechanical wash column 41 and brought into countercurrent contact with the crystal bed to wash the crystals. Also, the mother liquor in the wash column is returned to the ripening tank 21 through a line 75 that returns the mother liquor to the crystallization unit. The compound is thus purified into a high-purity compound.

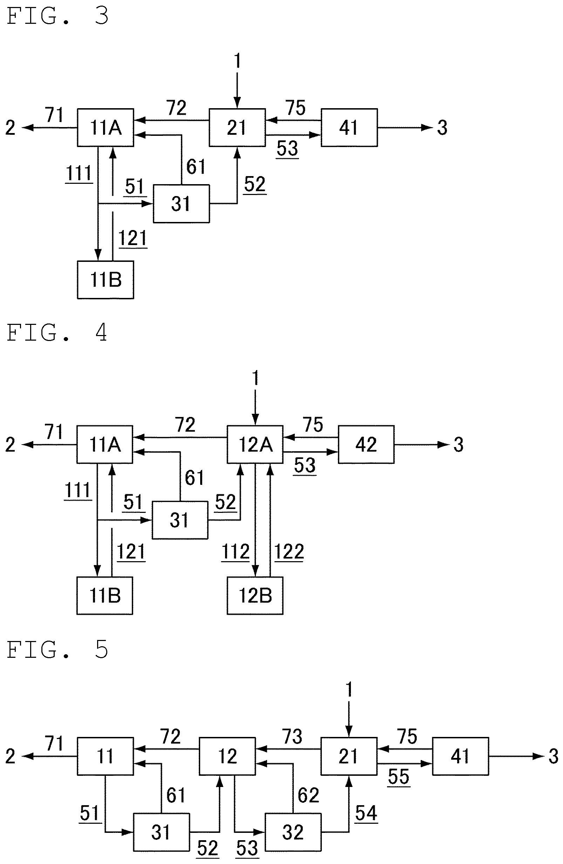

FIG. 3 illustrates an apparatus including a crystallization unit that includes one crystallization tank and one ripening tank. The apparatus includes a line that directly sends a mother liquor from the ripening tank to the next downstream crystallization tank, and a line that directly discharges the residue (mother liquor) from the crystallization tank, which is the most downstream tank. The crystallization tank is of a type that the contents of the tank are cooled outside the tank. The following describes only parts different from the purification apparatus of FIG. 2.

The crystallization tank 11 includes a tank 11A and a cooling mechanism 11B provided outside the tank, which are connected by lines 111 and 121. The solution of a compound (or the slurry containing crystals of a compound) sent from the tank 11A to the cooling mechanism 11B through the line 111 is cooled using the cooling mechanism 11B, and the slurry containing the precipitated crystals is sent to the tank 11A through the line 121. A portion of the slurry containing crystals of a compound is sent from the tank 11A to the cooling mechanism 11B through the line 111, and the rest of the slurry is sent to the solid-liquid separator 31 through the line 51.

FIG. 4 illustrates an apparatus including a crystallization unit that includes two crystallization tanks. The apparatus includes a line that directly sends a mother liquor from a crystallization tank 12 to the next downstream crystallization tank 11, and a line that directly discharges the residue (mother liquor) from the crystallization tank 11, which is the most downstream tank. The wash column is a hydraulic wash column and includes a mechanical mechanism that scrapes off the crystal bed. The following describes only parts different from the purification apparatus of FIG. 2.

The compound solution 1 to be fed to the purification apparatus is introduced into the crystallization tank 12. The crystallization tank 11 includes the tank 11A and the cooling mechanism 11B provided outside the tank, which are connected by the lines 111 and 121. The solution of a compound (or the slurry containing crystals of a compound) sent from the tank 11A to the cooling mechanism 11B through the line 111 is cooled using the cooling mechanism 11B, and the slurry containing the precipitated crystals is sent to the tank 11A through the line 121. A portion of the slurry containing crystals of a compound is sent from the tank 11A to the cooling mechanism 11B through the line 111, and the rest of the slurry is sent to the solid-liquid separator 31 through the line 51.

The crystallization tank 12 also includes a tank 12A and a cooling mechanism 12B provided outside the tank, which are connected by lines 112 and 122. A portion of the slurry containing crystals of a compound is sent from the tank 12A to the cooling mechanism 12B through the line 112, and sent back to the tank 12A through the line 122.

The crystal slurry is sent from the crystallization tank 12 to a hydraulic wash column 42 through the line 53. The crystal bed is scraped off in the lower part of the hydraulic wash column 42 using a mechanical mechanism (scraper), discharged while being suspended in a circulation liquid, and melted by heating to obtain a melt. A portion of the circulation liquid containing the melt is sent out as the high-purity compound 3. A portion of the rest of the circulation liquid (washing liquid) is returned to the hydraulic wash column 42 and brought into countercurrent contact with the crystal bed to wash the crystals.

FIG. 5 illustrates an apparatus including a crystallization unit that includes two crystallization tanks and one ripening tank. The apparatus includes lines each of which is disposed between any two adjacent tanks of the three tanks and directly sends a mother liquor from a tank one upstream, and a line that directly discharges the residue (mother liquor) from the most downstream tank. The following describes only parts different from the purification apparatus of FIG. 2.

The slurry containing crystals precipitated through cooling in the crystallization tank 11 that includes a cooling mechanism is sent to the solid-liquid separator 31 through the line 51. In the solid-liquid separator 31, the slurry is separated into a mother liquor and a concentrated crystal slurry. The concentrated crystal slurry is sent to the adjacent crystallization tank 12 through the line 52, and the mother liquor is returned to the crystallization tank 11 through the line 61. The residue 2 is discharged from the crystallization tank 11 through the line 71 to the outside of the purification apparatus. Thereby, the liquid level in the crystallization tank 11 is adjusted. The same operation as that performed in the crystallization tank 11 is performed in the crystallization tank 12, and the crystal-containing slurry is sent from the crystallization tank 12 to a solid-liquid separator 32 through the line 53. In the solid-liquid separator 32, the slurry is separated into a mother liquor and a concentrated crystal slurry. The concentrated crystal slurry is sent to the adjacent ripening tank 21 through a line 54, and the mother liquor is returned to the crystallization tank 12 through a line 62. In order to adjust the liquid level in the crystallization tank 12, the mother liquor is directly sent from the crystallization tank 12 to the crystallization tank 11 through the line 72. The crystals are grown in the ripening tank 21, and then, the crystal slurry is sent to the mechanical wash column 41 through a line 55. In order to adjust the liquid level in the ripening tank 21, the mother liquor is directly sent from the ripening tank 21 to the crystallization tank 12 through a line 73 that connects the ripening tank 21 to the crystallization tank 12.

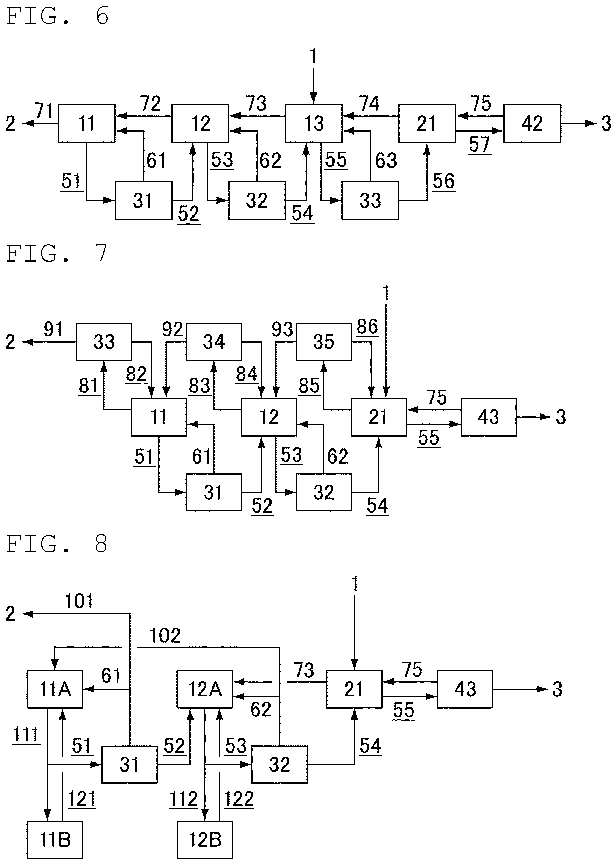

FIG. 6 illustrates an apparatus including a crystallization unit that includes three crystallization tanks and one ripening tank. The apparatus includes lines each of which is disposed between any two adjacent tanks of the four tanks and directly sends a mother liquor from a tank one upstream and a line that directly discharges the residue (mother liquor) from the most downstream tank. The wash column is a hydraulic wash column and has a mechanical mechanism that scrapes off the crystal bed. The following describes only parts different from the purification apparatus of FIG. 5.

The compound solution 1 to be fed to the purification apparatus is introduced into a crystallization tank 13. The crystal-containing slurry is sent to the solid-liquid separator 32 through the line 53 from the crystallization tank 12, which is the second most downstream tank from the most downstream tank. In the solid-liquid separator 32, the slurry is separated into a mother liquor and a concentrated crystal slurry. The concentrated crystal slurry is sent to the adjacent crystallization tank 13 through the line 54, and the mother liquor is returned to the crystallization tank 12 through the line 62. In order to adjust the liquid level in the crystallization tank 12, the mother liquor is directly sent from the crystallization tank 12 to the crystallization tank 11 through the line 72. The same operation as that performed in the crystallization tank 12 is performed in the crystallization tank 13, and the crystal-containing slurry is sent from the crystallization tank 13 to a solid-liquid separator 33 through the line 55. In the solid-liquid separator 33, the slurry is separated into a mother liquor and a concentrated crystal slurry. The concentrated crystal slurry is sent to the adjacent ripening tank 21 through a line 56, and the mother liquor is returned to the crystallization tank 13 through a line 63. In order to adjust the liquid level in the crystallization tank 13, the mother liquor is directly sent from the crystallization tank 13 to the crystallization tank 12 through the line 73. The crystals are grown in the ripening tank 21, and then, the crystal slurry is sent to the hydraulic wash column 42 through a line 57. In order to adjust the liquid level in the ripening tank 21, the mother liquor is directly sent from the ripening tank 21 to the crystallization tank 13 through a line 74 that connects the ripening tank 21 to the crystallization tank 13.

The crystal bed is scraped off in the lower part of the hydraulic wash column 42 using a mechanical mechanism (scraper), discharged while being suspended in a circulation liquid, and melted by heating to obtain a melt. A portion of the circulation liquid containing the melt is sent out as the high-purity compound 3. A portion of the rest of the circulation liquid (washing liquid) is returned to the hydraulic wash column 42 and brought into countercurrent contact with the crystal bed to wash the crystals.

FIG. 7 illustrates an apparatus including a crystallization unit that includes two crystallization tanks and one ripening tank. The apparatus includes lines each of which is disposed between any two adjacent tanks of the three tanks and sends a mother liquor from a tank one upstream via a solid-liquid separator, and a line that discharges the residue from the most downstream tank via a solid-liquid separator. The following describes only parts different from the purification apparatus of FIG. 5.

The crystallization tank 11 in FIG. 7 is provided with, instead of the line that directly discharges the residue, the solid-liquid separator 33 that separates the residue from the slurry in the crystallization tank and the line that, for example, discharges the residue through the solid-liquid separator 33. The slurry discharged from the crystallization tank 11 is sent to the solid-liquid separator 33 through a line 81, the residue 2 separated in the solid-liquid separator 33 is discharged to the outside of the purification apparatus through a line 91, and the remaining concentrated crystal slurry is returned to the crystallization tank 11 through a line 82. Thereby, the liquid level in the crystallization tank 11 is adjusted.

The crystallization tank 12 is provided with, instead of the line that directly sends a mother liquor to the crystallization tank 11, a solid-liquid separator 34 and a line that, for example, sends the mother liquor to the crystallization tank 11 through the solid-liquid separator 34. The slurry discharged from the crystallization tank 12 is sent to the solid-liquid separator 34 through a line 83, the mother liquor separated in the solid-liquid separator 34 is sent to the crystallization tank 11 through a line 92 to adjust the liquid level, and the remaining concentrated crystal slurry is returned to the crystallization tank 12 through a line 84.

The ripening tank 21 is provided with, instead of the line that directly sends a mother liquor to the crystallization tank 12, a solid-liquid separator 35 and a line that, for example, sends the mother liquor to the crystallization tank 12 through the solid-liquid separator 35. The slurry discharged from the ripening tank 21 is sent to the solid-liquid separator 35 through a line 85, the mother liquor separated in the solid-liquid separator 35 is sent to the crystallization tank 12 through a line 93 to adjust the liquid level, and the remaining concentrated crystal slurry is returned to the ripening tank 21 through a line 86.

The wash column 43 is a hydraulic wash column and includes no mechanical mechanism that scrapes off the crystal bed.

FIG. 8 illustrates an apparatus including a crystallization unit that includes two crystallization tanks and one ripening tank. The apparatus includes a line that sends a mother liquor from the second crystallization tank to the most downstream (first) crystallization tank via a solid-liquid separator, a line that discharges the residue from the most downstream tank via a solid-liquid separator, and a line that directly sends a mother liquor from the ripening tank to the second crystallization tank. The solid-liquid separator that separates a mother liquor from the slurry discharged from the second crystallization tank and sends the mother liquor to the most downstream crystallization tank and the solid-liquid separator that separates the residue to be discharged from the most downstream crystallization tank to the outside of the purification apparatus are each also used as a solid-liquid separator in a line that sends a slurry from a tank to the next upstream tank. The crystallization tank is of a type that the contents of the tank are cooled outside the tank. The following describes only parts different from the purification apparatus of FIG. 7.

In the apparatus of FIG. 8, the crystallization tank 11 includes the tank 11A and the cooling mechanism 11B provided outside the tank, which are connected by the lines 111 and 121. The solution of a compound (or the slurry containing crystals of a compound) sent from the tank 11A to the cooling mechanism 11B through the line 111 is cooled using the cooling mechanism 11B, and the slurry containing the precipitated crystals is sent to the tank 11A through the line 121. A portion of the slurry containing crystals of a compound is sent from the tank 11A to the cooling mechanism 11B through the line 111, and the rest of the slurry is sent to the solid-liquid separator 31 through the line 51. The crystallization tank 12 also includes the tank 12A and the cooling mechanism 12B provided outside the tank, which are connected by the lines 112 and 122. A portion of the slurry containing crystals of a compound is sent from the tank 12A to the cooling mechanism 12B through the line 112, and the rest of the slurry is sent to the solid-liquid separator 32 through the line 53.

In the apparatus of FIG. 8, a portion of the slurry containing crystals of a compound is sent from the tank 11A to the solid-liquid separator 31 through the line 51. In the solid-liquid separator 31, the slurry is separated into a mother liquor and a concentrated crystal slurry. The concentrated crystal slurry is sent to the adjacent tank 12A through the line 52. A portion of the mother liquor separated in the solid-liquid separator 31 is returned to the tank 11A through the line 61, and the rest of the mother liquor is discharged to the outside of the purification apparatus through an additional line 101 connected to the line 61. Also, a portion of the slurry containing crystals of a compound is sent from the tank 12A to the solid-liquid separator 32 through the line 53. In the solid-liquid separator 32, the slurry is separated into a mother liquor and a concentrated crystal slurry. The concentrated crystal slurry is sent to the adjacent ripening tank 21 through the line 54. A portion of the mother liquor separated in the solid-liquid separator 32 is returned to the crystallization tank 12 through the line 62, and the rest of the mother liquor is sent to the tank 11A through an additional line 102 connected to the line 62.

The flow from the line 51 to the solid-liquid separator 31 to the lines 61 and 101 in the purification apparatus of FIG. 8 corresponds to the flow from the line 81 to the solid-liquid separator 33 to the lines 82 and 91 in the apparatus of FIG. 7. The solid-liquid separator 31 in the line that sends a slurry to an upstream tank is also used as the solid-liquid separator 33. Also, the flow from the line 53 to the solid-liquid separator 32 to the lines 62 and 102 corresponds to the flow from the line 83 to the solid-liquid separator 34 to the lines 84 and 92 in the apparatus of FIG. 7. The solid-liquid separator 32 in the line that sends a slurry to an upstream tank is also used as the solid-liquid separator 34.

The apparatus of FIG. 8 includes the line 73 that directly sends a mother liquor from the ripening tank 21 to the tank 12A instead of the line that sends a mother liquor from the ripening tank 21 to the crystallization tank 12 via the solid-liquid separator 35 in the apparatus of FIG. 7.

### (Method for purifying easily polymerizable compound)

The present invention also relates to a method for purifying an easily polymerizable compound, the method using a purification apparatus including a wash column and at least one of a crystallization tank or a ripening tank, the crystallization tank forming easily polymerizable compound crystals from an easily polymerizable compound-containing solution or a mother liquor in an easily polymerizable compound crystal-containing slurry, the ripening tank growing the easily polymerizable compound crystals, the purification apparatus further including a line that includes a bend having a ratio R/D of radius R of curvature to line inner diameter D of more than 4 and not more than 10 and that is connected to at least one selected from the group consisting of the crystallization tank, the ripening tank, and the wash column, the method including sending the easily polymerizable compound crystal-containing slurry through the line.

The purification method of the present invention can efficiently purify an easily polymerizable compound to provide a high-quality product.

Preferred embodiments of the purification method of the present invention are the same as the preferred embodiments of the production method of the present invention described above.

### (Purification apparatus of the present invention)

The present invention also relates to an apparatus for purifying an easily polymerizable compound, the apparatus including a wash column and at least one of a crystallization tank or a ripening tank, the crystallization tank forming easily polymerizable compound crystals from an easily polymerizable compound-containing solution or a mother liquor in an easily polymerizable compound crystal-containing slurry, the ripening tank growing the easily polymerizable compound crystals, the purification apparatus further including a line that includes a bend having a ratio R/D of radius R of curvature to line inner diameter D of more than 4 and not more than 10 and that is connected to at least one selected from the group consisting of the crystallization tank, the ripening tank, and the wash column, the line being for sending an easily polymerizable compound crystal-containing slurry.

The line is not limited as long as it includes a bend that has a ratio R/D of radius R of curvature to line inner diameter D of more than 4 and not more than 10. Preferably, the line connects the crystallization tank or the ripening tank to the wash column.

In other words, preferably, in the purification apparatus of the present invention, the line connects the wash column to the crystallization tank or the ripening tank and is for sending the easily polymerizable compound crystal-containing slurry from the crystallization tank or the ripening tank to the wash column.

The line and other units in the purification apparatus of the present invention may be preferably those described above.

The crystallization tank may include a cooling mechanism and is not limited as long as it can cools the easily polymerizable compound-containing solution or the easily polymerizable compound crystal-containing slurry to precipitate and form crystals. Crystallization tanks including a cooling mechanism are roughly classified into a type where crystals are formed by directly cooling the inside of the tank with a cooling jacket directly attached to the tank (see FIGS. 2, 5, 6, and 7) and a type where crystals are formed by performing cooling in circulation where the tank and a cooling mechanism separated from the tank are connected with a pipe (see FIGS. 3, 4, and 8).

The cooling mechanism separated from the tank is not limited as long as it can cool the easily polymerizable compound-containing solution or the easily polymerizable compound crystal-containing slurry to precipitate crystals. Examples thereof include a cooling disc crystallizer, a scraped surface heat exchanger, and a Votator heat exchanger, each of which can ensure a large heat transfer area.

The crystallization temperature in the crystallization tank may be the temperature in the crystallizing described above.

The purification apparatus of the present invention has the crystallization tank and/or ripening tank, and may have one or more tanks. When the purification apparatus of the present invention includes multiple crystallization tanks (first to N-th tanks where the first tank is the most downstream tank and the N-th tank is the most upstream tank), these crystallization tanks are preferably connected in series. In this case, the purification apparatus of the present invention typically includes a line that sends an easily polymerizable compound crystal-containing slurry from one tank to another tank optionally via a solid-liquid separator. In this case, in the purification apparatus of the present invention, at least one tank includes a line that feeds an easily polymerizable compound-containing solution or an easily polymerizable compound crystal-containing slurry.

As described above, the purification apparatus of the present invention should have the crystallization tank and/or ripening tank. The purification apparatus may or may not have the ripening tank. Preferably, it has the ripening tank. The ripening tank in the present invention is a tank that grows crystals of a compound. The crystals are grown so as to have as uniform a crystal shape as possible before sending to the wash column. Thereby, impurities can be efficiently removed in the wash column and a high-purity compound can be obtained in high yield. Thus, a tank that sends liquid to the wash column, that is, the N-th tank, is preferably a ripening tank.

Most preferably, the purification apparatus of the present invention includes a line that sends the slurry containing crystals of a compound from the ripening tank to the wash column.

The ripening tank is not limited as long as it can keep the easily polymerizable compound crystals suspended in the tank. When the crystals are kept for a certain period of time, fine crystals are melted by Ostwald ripening and large crystals grow further, so that the crystal size distribution becomes narrow. As a result, high-quality crystals can be obtained, and by subjecting such crystals to the subsequent purification in the hydraulic wash column or the like, the purification efficiency in the hydraulic wash column can be further improved.

The temperature in the ripening tank may be the temperature in the above-described crystallizing.

The ripening tank typically includes, in the vicinity of the bottom, a discharge port that discharges the easily polymerizable compound crystal-containing slurry from the ripening tank.

The crystallization tank or the ripening tank may have any size. For example, each tank preferably has an inner diameter of 100 to 50000 mm. Also, each tank preferably has a height of 1000 to 100000 mm.

The purification apparatus of the present invention may further include an absorption column that collects an easily polymerizable compound-containing gas and provides an easily polymerizable compound-containing solution, and a line that sends the easily polymerizable compound-containing solution from the absorption column to the crystallization tank or the ripening tank.

The absorption column allows the reaction product, the easily polymerizable compound-containing gas, obtained in the reactor to be absorbed into water in which a polymerization inhibitor is dissolved, thereby providing the easily polymerizable compound-containing solution.

The line that sends the easily polymerizable compound-containing solution from the absorption column to the crystallization tank or the ripening tank may include an intermediate tank, a resin tank filled with an ion exchange resin, a low-boiling distillation apparatus, and the like. As described above, when the easily polymerizable compound is a (meth)acrylic acid, the low-boiling components such as acrolein, acetic acid, and water are removed by low-boiling distillation.

The purification apparatus of the present invention is preferably one capable of performing continuous purification. For example, the purification apparatus further includes a wash column (preferably a wash column that forcibly transports crystals) as a subsequent stage of the tank in the present invention.

The purification apparatus of the present invention may further include a line that discharges the circulation slurry containing crystals from the wash column, and a unit that melts the crystals in the discharged circulation slurry.

The crystals are derived from the crystal bed formed in the lower part of the wash column. The crystals can be discharged using a mechanism that discharges the crystals from the crystal bed in the wash column, for example.

The crystals are typically discharged together with the circulation liquid, i.e., the crystals are discharged in the form of a crystal-containing circulation slurry. This circulation slurry is fed to the melting step.

The purification apparatus of the present invention may further include: a line (discharge line) that connects the discharge port for crystals of the wash column to a melting unit; and a return line that connects a melting unit to a return port that is provided on the wash column and is for a circulation liquid containing the melt of the discharged crystals. A circulation liquid circulates through these lines and the connections between the return port and the crystal discharge port in the wash column. These circulation paths are also referred to as a melt loop.

The melting unit may be a heater. Examples of the heater include those having a structure that efficiently transfers heat to the crystal-containing slurry, such as a vertical multitubular heat exchanger, a horizontal multitubular heat exchanger, a double pipe heat exchanger, a spiral heat exchanger, a plate heat exchanger, and an electric heater. Preferably, the heater is provided in the melt loop, and the circulation slurry (the circulation liquid after melting) is circulated in the forced circulation system in which they are circulated by a pump provided in the melt loop.

The purification apparatus of the present invention may further include a line that is connected to the return line and sends out the product from the wash column.

The purification apparatus of the present invention may further include a line that returns the mother liquor from a tank or unit in the upstream side (the subsequent stage) to a tank or unit in the downstream side (the preceding stage).

The purification apparatus of the present invention may further include a mechanism that controls the amount of the slurry to be sent and the amount of the mother liquor to be returned. Examples of the control mechanism include valves attached to the lines.

The purification apparatus of the present invention may appropriately include different units commonly used in the purification apparatus.

### EXAMPLES

The present invention will be described in more detail below with reference to examples, but the present invention is not limited by the following examples, and appropriate modifications may be made within the scope that can conform to the gist of the above and later descriptions. All of them are included in the technical scope of the present invention.

Unless otherwise specified, "%" indicates "% by mass" and "parts" indicates "parts by mass."

### (Measurement instruments for gas chromatography and liquid chromatography)

Gas chromatograph: GC-2014 available from Shimadzu Corporation
High-performance liquid chromatography: LC-20AD HPLC unit available from Shimadzu Corporation
These instruments were used for measurement of furfural.

### (Method for preparing acrylic acid aqueous solution)

An acrylic acid aqueous solution was prepared according to the method described in WO 2010/032665 as follows: propylene was catalytically oxidized in vapor phase to obtain an acrylic acid-containing gas; and the acrylic acid-containing gas was treated in an absorption column.

### (Method for preparing feed slurry)

An acrylic acid aqueous solution was fed to a crystallization tank having an inner diameter of 650 mm. A cooling medium was fed to a jacket provided around the wall of the crystallization tank for indirect cooling. The resulting crystals attached to the inner surface of the crystallization tank were scraped off with a scraper provided in the crystallization tank. Thus, a crystal-containing slurry (feed slurry) was prepared.

### (Method for purifying slurry)

The wash column that purifies the feed slurry was a hydraulic wash column. The feed slurry was sent through a feed line described below and fed to the wash column, and while a mother liquor in the feed slurry was withdrawn through a filter in the wash column, the crystal bed formed in the column was moved to the lower part of the column by hydraulic pressure, and the crystals were melted in the melt loop. Thus, a product was obtained.

### <Purification apparatus and operation conditions>

The crystallization tank and the wash column prepared were connected by a feed line having an inner diameter of 25 mm. The feed line had three bends, each with an angle of 90°. The feed slurry prepared in the crystallization tank was sent through the feed line and fed to the wash column under the conditions of a slurry crystal concentration of 11.0% by mass and a slurry feed temperature of 10.5°C. Thereby, a product was obtained from the feed slurry. The feed line included a jacket as a heating mechanism, and a heating medium set at 15°C was introduced into the jacket.

### <Example 1>

Acrylic acid was obtained as a product under the conditions that the ratio R/D of radius R of curvature to line inner diameter D of each of the bends of the feed line was 5 and the linear velocity of the feed slurry was 1.5 m/s. Table 1 shows the concentration of furfural in the product, the furfural ratio, and the clogging state of the pipe.

### <Examples 2 and 3>

Acrylic acid was obtained as a product as in Example 1, except that the linear velocity of the feed slurry was changed as shown in Table 1. Table 1 shows the concentration of furfural in the product, the furfural ratio, and the clogging state of the pipe.

### <Example 4>

Acrylic acid was obtained as a product as in Example 1, except that the ratio R/D of radius R of curvature to line inner diameter D of each of the bends of the feed line was changed to 10 and the linear velocity of the feed slurry was changed to 1.8 m/s. Table 1 shows the concentration of furfural in the product, the furfural ratio, and the clogging state of the pipe.

### <Example 5>

Acrylic acid was obtained as a product as in Example 1, except that the linear velocity of the feed slurry was changed as shown in Table 1. Table 1 shows the concentration of furfural in the product, the furfural ratio, and the clogging state of the pipe.

### <Example 6>

Acrylic acid was obtained as a product as in Example 1, except that the crystal concentration of the feed slurry was changed as shown in Table 1. Table 1 shows the clogging state of the pipe.

### <Comparative Example 1>

Acrylic acid was obtained as a product as in Example 1, except that the ratio R/D of radius R of curvature to line inner diameter D of each of the bends of the feed line was changed to 3 and the linear velocity of the feed slurry was changed to 1.8 m/s. Table 1 shows the concentration of furfural in the product, the furfural ratio, and the clogging state of the pipe.

### <Example 7>

Acrylic acid was obtained as a product as in Example 1, except that the linear velocity of the feed slurry was changed as shown in Table 1. Table 1 shows the concentration of furfural in the product, the furfural ratio, and the clogging state of the pipe.

**[Table 1]**

| | R/D | Linear velocity of slurry | Crystal concentration of slurry | Concentration of furfural in product | Furfural ratio | Clogging of pipe |
|---|---|---|---|---|---|---|
| | [-] | [m/s] | [mass%] | [ppm] | [-] | |
| Example 1 | 5 | 1.5 | 11.0 | 0.20 | 1.67 | No clogging |
| Example 2 | 5 | 1.8 | 11.0 | 0.17 | 1.42 | No clogging |
| Example 3 | 5 | 2.3 | 11.0 | 0.12 | 1 | No clogging |
| Example 4 | 10 | 1.8 | 11.0 | 0.17 | 1.42 | No clogging |
| Example 5 | 5 | 3.2 | 11.0 | 0.16 | 1.33 | No clogging |
| Example 6 | 5 | 1.5 | 35.0 | - | - | No clogging |
| Comparative Example 1 | 3 | 1.8 | 11.0 | 0.43 | 3.58 | No clogging |
| Example 7 | 5 | 1.0 | 11.0 | 0.27 | 2.24 | No clogging |

| | | | | | | |
|---|---|---|---|---|---|---|
| * Furfural ratio is based on concentration of furfural in product of Example 3 | | | | | | |

As shown in Table 1 (Examples 2 and 4 and Comparative Example 1), when the R/D is small (4 or less), the concentration of furfural in the product increases and the purity of the product decreases. Table 1 also demonstrates that when the R/D is 5 or more, the concentration of furfural in the product is almost constant even when the R/D is increased. Therefore, as described above, the ratio R/D of radius R of curvature to line inner diameter D is preferably 10 or less. Increasing the R/D to 10 or more only increases the scale of the purification apparatus but cannot produce higher-quality acrylic acid.

The results shown in Table 1 (Examples 1, 3, 5, and 7) demonstrate that a high-quality product with a low furfural concentration can be efficiently obtained when at least the linear velocity of the feed slurry is within a specific range (1.5 m/s or more and 4.0 m/s or less).

The results shown in Table 1 (Examples 1 and 6) demonstrate that clogging of the bends of the pipes in the purification apparatus can be sufficiently prevented when at least the crystal concentration of the feed slurry is within a specific range (1% by mass or higher and 40% by mass or lower). Clogging is commonly less likely to occur at a lower crystal concentration of the slurry. Thus, there is no concern about clogging of the pipes at a crystal concentration of the feed slurry equal to or lower than 11.0% by mass, which is the crystal concentration of the feed slurry shown in Example 1.

These results demonstrate that a high-quality product can be obtained by a method for producing an easily polymerizable compound, the method using a purification apparatus including a tank(s) such as a crystallization tank and a wash column and including sending an easily polymerizable compound crystal-containing slurry through a line that is connected to the tank and/or wash column and includes a bend having a ratio R/D of radius R of curvature to line inner diameter D of more than 4 and not more than 10. The method is also capable of sufficiently preventing clogging of the line by crystals and efficiently providing a high-quality product. The reason why a high-quality product can be obtained by the above-described production method is that in the line, uneven crystal growth and crystal fracture are prevented to sufficiently prevent widening of the particle size distribution of the crystals, so that a crystal bed having a substantially uniform crystal shape can be formed in the wash column and the washability thereof is improved.

### REFERENCE SIGNS LIST

1: easily polymerizable compound-containing solution
2: residue
3: high-purity easily polymerizable compound
11, 12: crystallization tank including cooling mechanism
11A: tank (constituting most downstream crystallization tank (first crystallization tank))
11B, 12B: cooling mechanism (outside tank)
12A: tank (constituting second crystallization tank)
21: ripening tank
31 to 35: solid-liquid separator
41: mechanical wash column
42: hydraulic wash column (with mechanical mechanism that scrapes off crystal bed)
43: hydraulic wash column (without mechanical mechanism that scrapes off crystal bed)
51 to 57: line that sends slurry from downstream tank to upstream tank or wash column
61 to 63: line that returns mother liquor separated from slurry by solid-liquid separator to tank where slurry came from
71: line that directly discharges residue (mother liquor) from most downstream tank to outside of purification apparatus
72 to 74: line that directly sends mother liquor from upstream tank to next downstream tank
75: line that returns mother liquor from wash column to ripening tank
81 to 86: line that separates concentrated crystal-containing slurry by solid-liquid separator from slurry discharged from tank and returns slurry to tank where slurry came from
91: line that discharges residue (mother liquor) to outside of purification apparatus, the residue being separated by solid-liquid separator from slurry discharged from tank
92, 93: line that sends mother liquor to one- downstream tank, the mother liquor being separated by solid-liquid separator from slurry discharged from tank
101: additional line that discharges portion of mother liquor to outside of purification apparatus, the mother liquor being separated by solid-liquid separator from slurry discharged from most downstream tank
102: additional line that sends portion of mother liquor to most downstream tank, the mother liquor being separated by solid-liquid separator from slurry discharged from second tank
111, 112, 121, 122: line that connects cooling mechanism to tank of crystallization tank which cools contents of tank outside tank
D: line inner diameter
R: radius of curvature

## Claims

1. A method for producing an easily polymerizable compound, the method using a purification apparatus comprising a wash column and at least one of a crystallization tank or a ripening tank, the crystallization tank forming easily polymerizable compound crystals from an easily polymerizable compound-containing solution or a mother liquor in an easily polymerizable compound crystal-containing slurry, the ripening tank growing the easily polymerizable compound crystals,
the purification apparatus further comprising a line that includes a bend having a ratio R/D of radius R of curvature to line inner diameter D of more than 4 and not more than 10 and that is connected to at least one selected from the group consisting of the crystallization tank, the ripening tank, and the wash column,
the method comprising sending the easily polymerizable compound crystal-containing slurry through the line.

2. The method for producing an easily polymerizable compound according to claim 1,
wherein the line connects the wash column to the crystallization tank or the ripening tank, and
in the sending the slurry, the easily polymerizable compound crystal-containing slurry is sent from the crystallization tank or the ripening tank to the wash column.

3. The method for producing an easily polymerizable compound according to claim 1 or 2,
wherein in the sending the slurry, the slurry has a linear velocity of 1.5 m/s or more and 4.0 m/s or less.

4. The method for producing an easily polymerizable compound according to any one of claims 1 to 3,
wherein in the sending the slurry, the slurry has a crystal concentration of 1% by mass or higher and 40% by mass or lower.

5. The method for producing an easily polymerizable compound according to any one of claims 1 to 4,
wherein the wash column is a hydraulic wash column.

6. The method for producing an easily polymerizable compound according to any one of claims 1 to 5,
wherein the line includes a heating mechanism, and in the sending the slurry, the slurry has a temperature of 1°C or higher and 50°C or lower.

7. The method for producing an easily polymerizable compound according to any one of claims 1 to 6, further comprising:
obtaining an easily polymerizable compound-containing gas from a raw material; and
obtaining an easily polymerizable compound-containing solution from the easily polymerizable compound-containing gas.

8. The method for producing an easily polymerizable compound according to any one of claims 1 to 7,
wherein the easily polymerizable compound is a (meth)acrylic acid.

9. The method for producing an easily polymerizable compound according to claim 8,
wherein the (meth)acrylic acid is produced from at least one raw material selected from the group consisting of propane, propylene, acrolein, isobutene, methacrolein, acetic acid, lactic acid, isopropanol, 1,3-propanediol, glycerol, and 3-hydroxypropionic acid.

10. A method for purifying an easily polymerizable compound, the method using a purification apparatus comprising a wash column and at least one of a crystallization tank or a ripening tank, the crystallization tank forming easily polymerizable compound crystals from an easily polymerizable compound-containing solution or a mother liquor in an easily polymerizable compound crystal-containing slurry, the ripening tank growing the easily polymerizable compound crystals,
the purification apparatus further comprising a line that includes a bend having a ratio R/D of radius R of curvature to line inner diameter D of more than 4 and not more than 10 and that is connected to at least one selected from the group consisting of the crystallization tank, the ripening tank, and the wash column,
the method comprising sending the easily polymerizable compound crystal-containing slurry through the line.

11. An apparatus for purifying an easily polymerizable compound, the apparatus comprising:
a wash column; and
at least one of a crystallization tank or a ripening tank,
the crystallization tank forming easily polymerizable compound crystals from an easily polymerizable compound-containing solution or a mother liquor in an easily polymerizable compound crystal-containing slurry, the ripening tank growing the easily polymerizable compound crystals,
the purification apparatus further comprising a line that includes a bend having a ratio R/D of radius R of curvature to line inner diameter D of more than 4 and not more than 10 and that is connected to at least one selected from the group consisting of the crystallization tank, the ripening tank, and the wash column,
the line being for sending an easily polymerizable compound crystal-containing slurry.

12. The apparatus for purifying an easily polymerizable compound according to claim 11,
wherein the line connects the wash column to the crystallization tank or the ripening tank and is for sending the easily polymerizable compound crystal-containing slurry from the crystallization tank or the ripening tank to the wash column.
